(19) Europäisches Patentamt
European Patent Office
Office européen des brevets

(11) Veröffentlichungsnummer: **0 199 060**
**A2**

(12) **EUROPÄISCHE PATENTANMELDUNG**

(21) Anmeldenummer: **86103373.6**

(22) Anmeldetag: **13.03.86**

(51) Int. Cl.⁴: **C07F 9/58**, C07D 213/64, C07D 213/73, A01N 57/08

(30) Priorität: **03.04.85 DE 3512164**

(43) Veröffentlichungstag der Anmeldung: **29.10.86 Patentblatt 86/44**

(84) Benannte Vertragsstaaten: **AT BE CH DE FR GB IT LI LU NL SE**

(71) Anmelder: **CELAMERCK GmbH & Co. KG**
**Binger Strasse 173**
**D-6507 Ingelheim am Rhein(DE)**

(72) Erfinder: **Schröder, Ludwig, Dr. Dipl.-Chem.**
**Frankenstrasse 7**
**D-6507 Ingelheim am Rhein(DE)**
Erfinder: **Mengel, Rudolf, Dr. Dipl.-Chem.**
**Im Herzenacker 32**
**D-6535 Gau Algesheim(DE)**
Erfinder: **Drandarevski, Christo, Dr.**
**Boehringer Strasse 8**
**D-6507 Ingelheim am Rhein(DE)**

# (54) Pyridylphosphorsäureester.

(57) Die Erfindung betrifft neue Phosphorsäure-(thiophosphorsäure)ester, -thioester und -amide der Formel (I),

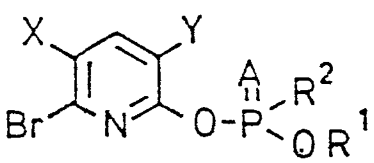

(I)

in der

$R^1$ für Alkylsteht,

$R^2$ für $OR^3$, $SR^3$ oder $NHR^3$ steht,

$R^3$ für Alkyl steht, A für O oder S steht und

X, Y, die gleich oder verschiedenen sein können für H, Cl oder Br stehen.

Verfahren zu deren Herstellung und ihre Verwendung als Schädlingsbekämpfungsmittel.

Des weiteren umfaßt die Verbindung neue unterschiedlich substituierte 6-Brom-pyridine, die als Ausgangsmaterialien zur Synthese von Verbindungen der Formel (I) Verwendung finden.

EP 0 199 060 A2

# Pyridylphosphorsäureester

Die Erfindung betrifft 6-Brompyridin-2-yl-phosphor (thiophosphor)säureester,

-thioester und -amide, Verfahren zu deren Herstellung und ihre Verwendung als Schädlingsbekämpfungsmittel.

Des weiteren umfaßt die Erfindung neue in Pos. 2 substituierte 6-Brompyridine, die als Zwischenproduckte zur Synthese der erfindungsgemäßen Phosphorsäurederivate Verwendung finden.

Es ist bekannt, daß bestimmte Pyridylphosphorsäureester bzw. thionophosphorsäureester, wie z.B. 0,0-Diethyl-0-(3,5,6-trichlor-2-pyridyl)-thionophosphorsäure (Chlorpyriphos) insektizid wirksam sind (vgl. DE-AS 14 45 659). Wirkung und Anwendungsbreite dieser Verbindungen sind jedoch nicht immer voll zufriedenstellend.

Es wurden nun neue 6-Brompyridin-2-yl phosphorsäureester der Formel (I) gefunden,

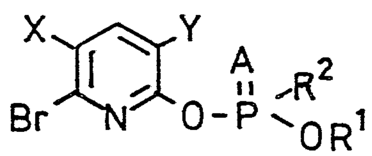

(I)

in der

$R^1$ für $C_1$ bis $C_4$-Alkyl steht,

$R^2$ für $OR^3$, $SR^3$ oder $NHR^3$ steht,

$R^3$ für $C_1$ bis $C_4$-Alkyl steht,

A für O oder S steht und

X, Y, die gleich oder verschieden sein können für H, Cl oder Br stehen.

Verbindungen der Formel (I) können hergestellt werden durch Umsetzung von 6-Brom-2-hydroxypyridinen der Formel (II)

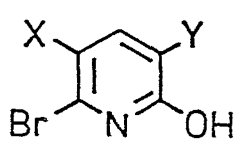

(II)

oder entsprechender Alkali-, Erdalkali-oder Ammoniumsalze mit Halogeniden der Formel (III)

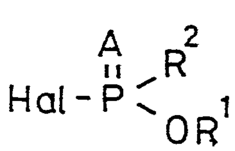

(III)

in der Hal für Halogen -vorzugsweise Chlor oder Brom -steht und die Reste A, $R^1$ und $R^2$ wie zuvor definiert sind. Die Reaktion wird gegebenenfalls in Gegenwart eines Säureakzeptors und gegebenenfalls in Gegenwart eines Verdünnungsmittels durchgeführt.

Des weiteren betrifft die Erfindung 6-Brom-2-hydroxypyridine der Formel (III),

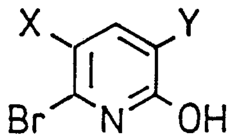

(II)

in der X, Y, die gleich oder verschieden sein können und für H, Cl oder Br stehen mit der Einschränkung, daß X und Y nicht gemeinsam für H oder Cl stehen.

Außerdem betrifft die Erfindung 2-Amino-6-brom-3,5-dihalogenpyridine der Formel (IV) - (X = Y = Cl, Br),

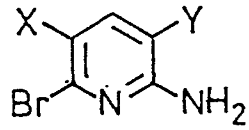

(IV)

Die vorstehend genannten Verbindungen der Formel (II) und (IV) sind neu und können als Ausgangsverbindungen zur Synthese der Phosphorsäureester (I) verwendet werden.

Die erfindungsgemäßen Verbindungen der Formel (I) haben bei hoher und anhaltender Wirkung als Schädlingsbekämpfungsmittel -insbesondere als akarizide, insektizide und nematizide Mittel - eine deutlich verringerte Warmblütertoxizität gegenüber den aus dem Stand der Technik bekannten strukturell ähnlichen Verbindungen.

Die Alkylreste $R^1$ und $R^3$ können verzweigt oder unverzweigt sein und stehen für Methyl, Ethyl, Propyl, Isopropyl, n-Butyl, sek-Butyl, iso-Butyl oder tert-Butyl.

Halogen steht für Fluor, Chlor, Brom oder Jod; Chlor und Brom sind bevorzugt.

Hervorzuheben sind Verbindungen der Formel I, in der X und Y gleich sind und für Wasserstoff, Chlor oder Brom stehen, wobei die Reste $R^1$ und $R^3$, die gleich oder verschieden sein können die zuvor genannte Bedeutung haben.

Wenn A für Schwefel steht oder wenn $R^2$ die Bedeutung $SR^3$ hat, können Verbindungen der formel I auch als Isomerengemisch vorliegen, in dem der Schwefel in den durch die Substituenten A, $SR^3$ und $R^1$ bezeichneten Positionen auftritt. Diese Isomerisierungen sind in der phosphororganischen Chemie bekannt. Die allgemeine Formel I umschließt somit auch die zuvor genannten Konstitutionsisomere.

Des weiteren sind Verbindungen der Formel I hervorzuheben, in denen X und Y gleich oder verschieden voneinander sind, die Rest $R^1$ und $R^3$ jedoch die gleiche Bedeutung haben und für Methyl, Ethyl, n-Propyl, iso-Propyl, n-Butyl, sek-Butyl, iso-Butyl oder tert-Butyl stehen.

Besonders bevorzugt sind nachstehend genannte Verbindungen der Formel (I):

Thiophosphorsäure-0-(6-brom-3,5-dichlorpyridin-2-yl-0,0-diethylester

Thiophosphorsäure-0-(6-brom-3,5-dichlorpyridin-2-yl)-0,0-dimethylester

Phosphorsäure-0-(6-brom-3,5-dichlorpyridin-2-yl)-0,0-diethylester

Thiophosphorsäure-0-(6-brom-3,5-dichlorpyridin-2-yl)-0-ethylisopropylamid.

Thiophosphorsäure-0-(6-brom-3,5-dichlorpyridin-2-yl)-0-ethyl-sek-butylamid,

Thiophosphorsäure-0-(6-brom-3,5-dichlorpyridin-2-yl),-0,0-diisopropylester

Thiosphosphorsäure-0-(6-brom-3,5-dichlorpyridin-2-yl),-0,S-diethylester

Thiophosphorsäure-0-(6-brom-3,5 dichlorpyridin-2-yl)-0-ethyl-S-propylester

Thiophosphorsäure-0,0-diethyl-0-(3,5,6-tribrompyridin-2-yl) ester

Thiophosphorsäure-0,0 diethyl-0-(6-brom-pyridin-2-yl)-ester

Thiophosphorsäure-0,0-dimethyl-0-(3,5,6-tribrom-pyridin-2-yl) ester

Thiophosphorsäure-0,0-dimethyl-0-(3,5,6-tribrompyridin-2-yl)-ester

Die Synthese der erfindungsgemäßen Verbindungen kann nach an sich bekannten Verfahren ausgehend von 2-Hydroxypyridinen der Formel (II) und Halogeniden der Formel (III) gemäß nachstehendem Formelschema durchgeführt werden:

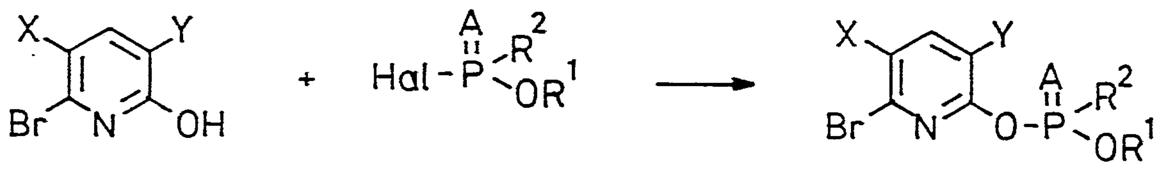

(II) (III) (I)

Anstelle der in Formel II angegebenen 2-Hydroxypyridine können auch die Salze dieser Verbindungen eingesetzt werden; bevorzugt sind die Alkali, Erdalkali oder Ammoniumsalze.

Verbindungen der Formel II sind:

6-Brom-2-hydroxypyridin,

2-Hydroxy-3,5,6-tribrompyridin,

5,6-Dibrom-2-hydroxypyridin,

3,6-Dibrom-2-hydroxypyridin,

6-Brom-5-chlor-2-hydroxypyridin,

6-Brom-3-chlor-2-hydroxypyridin,

3-Chlor-5,6-dibrom-2-hydroxypyridin

5-Chlor-3,6-dibrom-2-hydroxypyridin sowie das

6-Brom-3,5-dichlor-2-hydroxypyridin

. Von den 6-Brom-2-hydroxypyridinen der Formel (II) ist bisher nur das 6-Brom-2-hydroxypyridin (den Hertog, Rec. trav. Chim. Pay Bas; 55(1936) 122, 128) und das 6-Brom-3,5-dichlor-2-hydroxypyridin (US 41 15 557) beschrieben.

Die in den Positionen 3 und 5 ein-oder zweifach mit Chlor oder Brom substituierten bzw. unsubstituierten Pyridine der Formel (II) können z.B. durch Halogenierungsreaktionen ausgehend von 6-Brom-2-hydroxypyridinen der Formel II oder ausgehend von 6-Brom-2-aminopyridinen der Formel IV hergestellt werden, wobei in beiden Fällen X oder Y für Wasserstoff oder aber X und Y für Wasserstoff stehen.

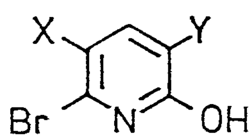

(II)

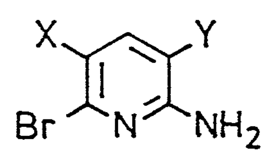

(IV)

Halogenierungsreaktionen von 2-Amino-bzw. 2-Hydroxypyridinen sind in der Literatur beschrieben.

Durch Variation der Reaktionsbedingungen kann die Halogenierung so geführt werden, daß selektiv die Positionen 3 bzw. 5 des Pyridinsystems mono-bzw. disubstituiert werden. Teilweise entstehen bei den Halogenierungsreaktionen Stellungsisomere, die nach an sich bekannten Methoden auftrennbar sind.

Ein weiterer Weg zur gezielten Synthese von 2-Hydroxy-pyridinen der Formel (II) besteht in der stufenweise Diazotiérung von 2,6-Diaminopyridinen der Formel V:

Aufgrund eigener Untersuchungen wurde festgestellt, daß 2,6-Diamino-3,5-dichlor-pyridin (V) bei der Diazotierung in wäßriger 60 %-HBr-Lösung überwiegend zum 2-Amino-6-brom-3,5-dichlorpyridin (IV) reagiert und nicht zum 2,6-Dibrom-3,5-dichlorpyridin (VI), wie in der Literatur angegeben - (J.C.S. Chem. Commun.; (1980) 1139).

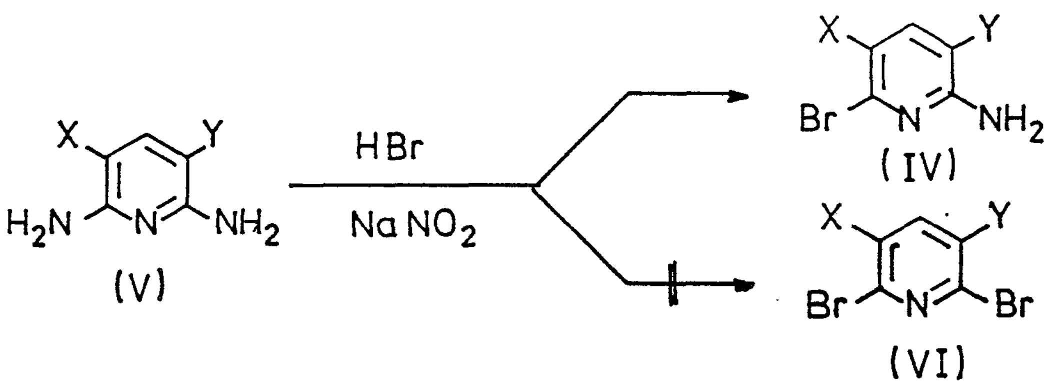

(V) (IV) (VI)

X = Y = Cl

2,6-Diaminopyridine der Formel (V), in der X bzw. Y für Chlor, Brom oder Wasserstoff stehen sind literaturbekannt: z.B. 3-Brom-2,6-diaminopyridin und 3-Chlor-2,6-diaminopyridin (M. Boudakian; J. Fluorine Chem,; 18 497) oder das 3-Brom-5-chlor-2,6-diaminopyridin (J.C.S. Chem. Commun.; 1980 1139).

Im Falle der zuvorgenannten unsymmetrisch substituierten Pyridine der Formel (V) kann das angestrebte Substitutionsmuster des 6-Brom-2-hydroxypyridins (II) gesteuert werden durch die Abfolge der Einzelschritte, d.i. Einführung der Hydroxyfunktion durch Diazotierung in verdünnter Säure und Einführung des 6-Brom-Substituenten durch Diazotierung von (V) in Bromwasserstoffsäure, oder durch eine Umkehrung dieser Einzelschritte.

Das erfindungsgemäße Verfahren zur Herstellung der neuen Phosphorsäureester der Formel (I) wird bevorzugt unter Verwendung von Verdünnungsmitteln durchgeführt. Als Verdünnungsmittel kommen praktisch alle inerten organischen Lösungsmittel infrage, des weiteren kann die Umsetzung auch in Wasser sowie in wäßriger Suspension zusammen mit einem inerten organischen Lösungsmittel als Cosolvens -vorzugsweise Toluol -durchgeführt werden.

Zu nennen sind insbesondere aliphatische und aromatische, gegebenenfalls halogenierte Kohlenwasserstoffe, wie Pentan, Hexan, Heptan, Cyclohexan, Petrolether, Benzin, Ligroin, Benzol. Toluol, Xylol. Methylenchlorid, Ethylenchlorid, Chloroform, Tetrachlorkohlenstoff, Chlorbenzol und o-Dichlorbenzol, Ether wie Diethyl-und Dibutylether, Glycoldimethylether und Diglycoldimethylether, Tetrahydrofuran und Dioxan, Ketone, wie Aceton, Methylethyl-, Methylisopropyl-und Methylsiobutylketon, Ester, wie Essigsäuremethylester und -ethylester, Nitrile, wie Acetonitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Das Verfahren kann gegebenenfalls in Gegenwart von Säureakzeptoren durchgeführt werden. Als Säureakzeptoren können alle üblichen Säurebindemittel Verwendung finden. Besonders bewährt haben sich Alkalicarbonate und -alkoholate, wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin sowie Alkalihydroxide und Erdalkalihydroxide sowie Erdalkalicarbonate.

Das erfindungsgemäße Verfahren wird im allgemeinen bei Temperaturen zwischen 0°C und 120°C durchgeführt. Bevorzugt wird der Bereich zwischen 20°C und 100°C. Die Umsetzungen werden im allgemeinen bei Normaldruck durchgeführt.

Zur Durchführung des erfindungsgemäßen Verfahrens werden die Ausgangsstoffe (II) und (III) gewöhnlich annähernd in äquimolaren Mengen eingesetzt. Ein Überschuß der einen oder anderen Reaktionskomponente bringt keine wesentlichen Vorteile.

Die Umsetzungen werden im allgemeinen in einem geeigneten Verdünnungsmittel und gegebenenfalls in Gegenwart eines Säureakzeptors durchgeführt. Die Aufarbeitung geschieht nach üblichen Methoden. Die neuen Verbindungen fallen zum Teil in Form von Oelen an, die sich zum Teil nicht unzersetzt destillieren lassen, jedoch durch sogenanntes "Andestillieren", d.h. längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechnungsindex und im Falle kristallisierender Produkte der Schmelzpunkt oder der dünnschicht chromatographisch bestimmbare Rf-Wert.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder - schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cylohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid, als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage:

z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel, als Emulgier-und/oder schaumerzeugende Mittel kommen in Frage:

z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage:

z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum,

Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Kohlenwasserstoffe, Phenacylharnstoffe, Pyrethroide, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Formulierungsbeispiele

a) Emulsionskonzentrate

20 Gew.-Teile eines Wirkstoffs gemäß der Erfindung werden in 75 Gew.-Teilen Xylol gelöst und mit 5 Gew.-Teilen Nonylphenolpolyglykoläther versetzt. Diese Lösung wird für die Anwendung mit Wasser emulgiert. Der Wirkstoffgehalt der Emulsion beträgt im allgemeinen zwischen 0,01 und 0,1 Gew.-%.

b) Stäubemittel

2 Gew.-Teile eines Wirkstoffs gemäß der Erfindung werden auf 98 Gew.-Teile Kaolin aufgedüst und mit diesem homogen vermahlen.

c) Suspensionspulver

25 Gew.-Teile eines erfindungsgemäßen Wirkstoffs werden auf 75 Gew.-Teile Kieselgur aufgedüst und nach Zusatz von 2 Teilen Natrium-naphthalinsulfonat homogen vermahlen. Für die Anwendung wird das erhaltene Suspensionspulver mit Wasser bis zu gewünschten Konzentration (vorzugsweise 0,01 -0,1 Gew.-% Wirkstoff) vermischt.

Beispiel 1

Herstellung einer Ausgangsverbindung der Formel (II) durch Halogenieren eines Aminopyridins der Formel (IV) und anschließende Diazotierung zu (II).

1a) 2-Amino-6-brom-3,5-dichlorpyridin (IV, X=Y=Cl) aus 2-Amino-6-brom-pyridin (IV, X=Y=H).

Zu einer Lösung von 173 g 2-Amino-6-brompyridin (IV) in 500 ml konz. Salzsäure werden innerhalb von 45 min. 74,8 g einer 30 %igen $H_2O_2$-Lösung getropft, wobei die Innentemperatur bei etwa 20°C gehalten wird. Danach wird noch 12 Stunden bei 20°C belassen und dann von dem ausgefallenen Produkt abgesaugt.

Ausbeute: 217,8 g (90%) 2-Amino-6-brom-3,5-dichlorpyridin vom Schmp. 147-148°C - (aus Cyclohexan).

1b) 6-Brom-3,5-dichlor-2-hydroxy-pyridin (II, X=Y=Cl) aus 2-Amino-6-brom-3,5-dichlorpyridin (IV, X=Y=Cl)

Zu einer Lösung von 24,2 g 2-Amino-6-brom-3,5-dichlorpyridin (IV) in 80 ml conc. Schwefelsäure und 20 ml Wasser werden bei 80°C 7,6 g $NaNO_2$ portionsweise zugegeben. Nach Abklingen der Stickstoffentwicklung wird das Reaktionsgemisch auf Raumtemperatur gekühlt, dann in 500 ml Eiswasser eingegossen und das ausfallende Produkt abgesaugt.

Ausbeute: 19,8 g (81,5 %) 6-Brom-3,5-dichlor-2-hydroxypyridin vom Schmp. 177-180°C.

Beispiel 2

Herstellung von Ausgangsverbindungen der Formel (II), durch Halogenierung von 6-Brom-2-hydroxypyridin (II, X=Y=H), ausgehend von 2-Amino-6-brompyridin (IV).

Zu einer Lösung von 34,6 g 2-Amino-6-brompyridin (IV, X=Y=H) in 200 ml 30 % Schwefelsäure wird unter Kühlen eine Lösung von 14,8 g $NaNO_2$ in 50 ml Wasser getropft. Dann wird noch 30 min. bei Raumtempertur nachgerührt. Zu dieser Suspension von 6-Brom-2-hydroxpyridin (II, X=Y=H) werden 200 ml konz. Salzsäure gegeben und bei 40°C 34 g einer 30 %igen $H_2O_2$-Lösung getropft. Nach Abklingen der exothermen Reaktion wird gekühlt und von dem ausfallenden Produkt abgesaugt.

Ausbeute: 41,2 g (84,8 %) 6-Brom-3,5-dichlor-2-hydroxy-pyridin (II) als graues Pulver vom Schmp. 179-185°C.

Analog kann 2-Hydroxy-3,5,6-tribrompyridin (II, X=Y=Cl) in 82 % Ausbeute hergestellt werden.

2-Hydroxy-3,5,6-tribrompyridin hat einen Schmp. von 190-192°C.

Beispiel 3

Umsetzung von 6-Brom-2-hydroxypyridinen der Formel (II) mit Halogeniden der Formel (III).

Thiophosphorsäure-0-(6-brom-3,5-dichlorpyridin-2-yl)-0,0-diethylester.

24,3 g 6-Brom-3,5-dichlor-2-hydroxypyridin (darstellbar nach Beispiel 1 bzw. 2) werden in 150 ml Aceton gelöst und mit 15,2 g fein gepulvertem $K_2CO_3$ versetzt, wobei sich das Kaliumsalz als voluminöser Niederschlag abscheidet. Es wird noch 15 min. bei Raumtemperatur nachgerührt und dann rasch mit 18,9 g 0,0-Diethyl-thiophosphorsäurechlorid versetzt und 2,5 Stunden zum Sieden erhitzt.

Danach wird von den anorganischen Fällungen abfiltriert, nachgewaschen und die Mutterlauge wird eingeengt. Es verbleiben 35 g eines dunklen Öls, das an Kieselgel mit Essigster/Heptan gereinigt wird. Die Hauptfraktion kristallisiert beim Anreiben mit Hexan.

Ausbeute: 30,9 g (89 %) Thiophosphorsäure-0-(6-brom-3,5-dichlorpyridin-2-yl)-0,0-diethylester als farblose Kristalle vom Schmp. 45-47°C.

Analog des in Beispiel 3 beschriebenen Verfahrens werden die in nachstehender Tabelle aufgeführten Verbindungen erhalten:

Tabelle: Verbindungen der Formel

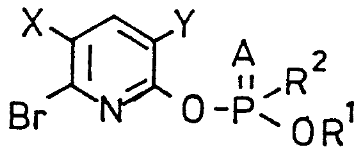

(I)

| Beispiel No. | A | X | Y | $R^1$ | $R^2$ | |
|---|---|---|---|---|---|---|
| 4 | S | Cl | Cl | $CH_3$ | $OCH_3$ | Fp: 44-46°C |
| 5 | O | Cl | Cl | $C_2H_5$ | $OC_2H_5$ | Fp: 39-42°C |
| 6 | S | Cl | Cl | $C_2H_5$ | $NHCH(CH_3)_2$ | Fp: 59-63°C |
| 7 | S | Cl | Cl | $C_2H_5$ | $NHCHCH_3(C_2H_5)$ | Fp: 47-51°C |
| 8 | S | Cl | Cl | $CH(CH_3)_2$ | $OCH(CH_3)_2$ | Öl |
| 9 | O | Cl | Cl | $C_2H_5$ | $SC_3H_7$ | Öl $n_D^{20}$: 1,5392 |
| 10 | S | Br | Br | $C_2H_5$ | $OC_2H_5$ | Fp: 65-66°C |
| 11 | S | H | H | $C_2H_5$ | $OC_2H_5$ | $n_D^{20}$: 1,5380 |

Beispiel A:

Spodoptera litura-Test

In 9-er-Töpfen herangezogene Vicia-Pflanzen - (Größe 20-25 je 5 Pflanzen/Topf) werden mit je 50 ml der angesetzten Spritzbrühen in der Spritzkabine tropfnaß behandelt. Nach Abtrocknen des Spritzbelages werden die Töpfe bis zu den Pflanzenstengeln in Plastiktüten eingepackt.

Danach werden die Vicia-Pflanzen mit je 10 Spodoptera-Raupen pro Topf besidelt.

Die Kontrolle der Mortalität der Raupen erfolgt am 1. und am 4. Tag nach der Besiedlung.

Bei diesem Test zeigen z.B. bei einer Wirkstoffkonzentration von weniger als 150 ppm die Verbindungen der Herstellungsbeispiele (3) und - (4) nach 4 Tagen eine Abtötung von 100 %.

**Ansprüche**

1) 6-Brompyridin-2-yl phosphorsäureester der Formel (I)

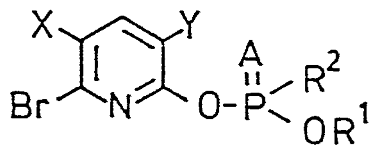

(I)

in der

$R^1$ für $C_1$ bis $C_4$-Alkyl steht,

$R^2$ für $OR^3$, $SR^3$ oder $NHR^3$ steht,

$R^3$ für $C_1$ bis $C_4$-Alkyl steht,

A für O oder S steht und

X, Y, die gleich oder verschieden sein können für H, Cl oder Br stehen.

2) Verbindungen der Formel (I) nach Anspruch 1, in der

X und Y gleich sind und für Wasserstoff, Chlor oder Brom stehen,

$R^1$ für $C_1$ bis $C_4$-Alkyl steht,

$R^2$ für $OR^3$, $SR^3$ oder $NHR^3$ steht,

$R^3$ für $C_1$ bis $C_4$-Alkyl steht,

und

A für O oder S steht.

3) Verbindungen der Formel (I) nach Anspruch 1, in der

X, Y, die gleich oder verschieden sein können, für H, Cl oder Br stehen,

$R^2$ für $OR^3$, $SR^3$ und $NHR^3$ steht und

$R^1$, $R^3$ die gleiche Bedeutung haben und für Methyl, Ethyl, n-Propyl, iso-Propyl, n-Butyl, sek-Butyl, iso-Butyl oder tert-Butyl stehen.

4) Thiophosphorsäure-0-(6-brom-3,5-dichlorpyridin-2-yl)-0,0-diethylester

5) Thiophosphorsäure-0-(6-brom-3,5-dichlorpyridin-2-yl)-0,0-dimethylester

6) Thiophosphorsäure-0-(3,5,6-tribrompyridin-2yl)-0,0-diethylester

7) 6-Brom-2-hydroxypyridine der Formel (II)

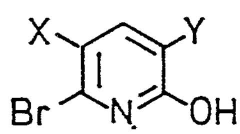

(II)

in der

X, Y, die gleich oder verschieden sein können für H, Cl oder Br stehen, mit der Einschränkung, daß X und Y nicht gemeinsam für Chlor oder Wasserstoff stehen.

8) Verfahren zur Herstellung von Verbindungen nach Anspruch 1 bis 5,

dadurch gekennzeichnet,

daß man ein 6-Brom-2-hydroxypyridin der Formel -(II)

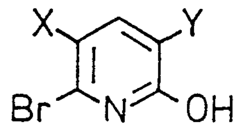 (II)

in der X, Y, die gleich oder verschieden sein können und für H, Cl oder Br stehen, oder Alkali-, Erdalkyli-oder Ammoniumsalze von Verbindungen der Formel (II) mit Halogeniden der Formel (III)

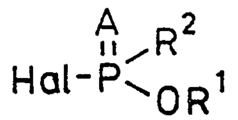 (III)

in der

$R^1$ für $C_1$ bis $C_4$-Alkyl

$R^2$ für $OR^3$, $SR^3$ oder $NHR^3$und $R^3$ für $C_1$ bis $C_4$-Alkyl steht,

gegebenenfalls in Gegenwart eines Säureakzeptors und gegebenenfalls in Gegenwart eines Verdünnungsmittel umsetzt.

9) Schädlingsbekämpfungsmittel gekennzeichnet durch einen Gehalt an mindestens einer Verbindung der Formel (I) nach Anspruch 1 bis 5.

10) Verwendung von Verbindungen der Formel (I) gemäß Anspruch 1 bis 5 zur Bekämpfung von Schädlingen insbesondere Insekten, Akariden oder Nematoden.

11) Verfahren zur Bekämpfung von Schädlingen,

dadurch gekennzeichnet,

daß man Verbindungen der Formel (I) nach Anspruch 1 bis 5 auf die Schädlinge, vorzugsweise Insekten, Akariden oder Nematoden oder ihren Lebensraum einwirken läßt.

12) Verfahren zur Herstellung von Schädlingsbekämpfungsmitteln,

dadurch gekennzeichnet,

daß man Verbindungen der Formel (I) nach Anspruch 1 bis 5 mit Streckmitteln und/oder oberflächenaktiven Mitteln vermischt.

13) Verwendung von Verbindungen der Formel (II) nach Anspruch 6 zur Synthese von Verbindungen der Formel (I) nach einem der Ansprüche 1 bis 3.

14) 2-Amino-6-brom-3,5-dichlor-pyridin.

15) Verwendung von 2-Amino-6-brom-3,5-dichlorpyridin zur Synthese von 6-Brom-3,5-dichlor-2-hydroxypyridin.

16) 2-Amino-3,5,6-tribrompyridin

17) Verwendung von 2-Amino-3,5,6-tribrompyridin zur Synthese von 2-Hydroxy-3,5,6-tribormpyridin.